Europäisches Patentamt

European Patent Office

Office européen des brevets

(11) Numéro de publication: **0 436 747 A1**

# DEMANDE DE BREVET EUROPEEN

(21) Numéro de dépôt: 89402431.4

(22) Date de dépôt: 06.09.89

(51) Int. Cl.5: **C12P 21/06, A61K 37/18, A23J 1/06**

(43) Date de publication de la demande:
17.07.91 Bulletin 91/29

(84) Etats contractants désignés:
**AT BE CH DE ES GB GR IT LI LU NL SE**

(71) Demandeur: **CENTRE NATIONAL DE LA RECHERCHE SCIENTIFIQUE (CNRS)**
**15, Quai Anatole France**
**F-75007 Paris(FR)**

Demandeur: **Etablissements Public dit:**
**UNIVERSITE DES SCIENCES ET TECHNIQUES (LILLE I)**
**Domaine Universitaire Scientifique**
**F-59655 Villeneuve d'Ascq Cédex(FR)**

(72) Inventeur: **Guillochon, Didier, Gérard, Daniel**
**70 Allée Chantecler**
**F-59650 Villeneuve d'Ascq(FR)**
Inventeur: **Piot, Jean-Marie, Edmond, Marcel**
**5 Avenue du vieux château**
**F-59650 Villeneuve d'Ascq(FR)**
Inventeur: **Leconte, Danielle Georgette Yvette**
**31/11 rue Baudoin IX**
**F-59650 Villeneuve d'Ascq(FR)**
Inventeur: **Thomas, Daniel**
**B.P. 233, rue Roger Couttolenc**
**F-60206 Compiegne Cédex(FR)**

(74) Mandataire: **Bérogin, Francis et al**
**c/o CABINET HARLE & PHELIP 21 rue de la Rochefoucauld**
**F-75009 Paris(FR)**

(54) Procédé d'obtention de fractions peptidiques héminiques issues d'hémolysats du cruor, les fractions obtenues et leurs applications.

(57) On conduit une hydrolyse enzymatique d'un hémolysat du cruor avec une enzyme protéolytique travaillant à pH <4 (pepsine) ; on soutire en continu le produit de réaction en l'amenant dans une zone d'ultrafiltration (7) comportant des membranes minérales d'ultrafiltration (8) ayant un seuil de coupure leur permettant de retenir ladite enzyme et l'hémoglobine ; on soutire en continu un perméat qui représente une fraction recherchée, soluble, ayant une teneur en fer $\geq$ 0,25 mg ou 0,50 mg par g de m. s. respectivement avant et après dessalage ; on recueille le rétentat qui représente une fraction recherchée, insoluble, dont la teneur en fer est $\geq$ 1% en poids des m. s. Ce rétentat peut être précipité à pH acide ou centrifugé, conduisant à des fractions héminiques soluble et insoluble, ou bien être soumis à une hydrolyse enzymatique (Alcalase), avec ultrafiltration, donnant un rétentat précipité à pH acide. La teneur en fer de la fraction précipitée est $\geq$ 3% en poids des m. s.

Fig 1

## PROCEDE D'OBTENTION DE FRACTIONS PEPTIDIOUES HEMINIQUES ISSUES D'HEMOLYSATS DU CRUOR, LES FRACTIONS OBTENUES ET LEURS APPLICATIONS.

La présente invention porte sur l'obtention de nouvelles fractions peptidiques héminiques issues de produits de l'hémolyse de la partie du sang qui est obtenue après séparation du plasma, cette partie étant désignée dans ce qui suit par le terme «cruor». Ces fractions, comportant du fer héminique doué d'une grande biodisponibilité, peuvent être classées en fractions solubles dans les milieux aqueux dans les conditions physiologiques (désignées ci-après simplement par l'expression «fractions peptidiques héminiques solubles»), et en fractions peptidiques héminiques insolubles, lesquelles présentent toutes deux une utilité dans des applications thérapeutiques nécessitant un apport en fer. Les premières, bien qu'appauvries en hème, en comportent suffisamment pour présenter de l'intérêt en thérapeutique, car leur propriété de solubilité les rend rapidement assimilables par l'organisme. Quant aux secondes, elles présentent de l'intérêt compte tenu de leur teneur élevée en fer.

La présente invention concerne donc un procédé pour l'obtention de ces deux types de fractions, ce procédé mettant en jeu l'hydrolyse en continu du cruor dans un réacteur enzymatique à ultrafiltration, le perméat d'ultrafiltration ou ultrafiltrat constituant, tel qu'il est obtenu, la fraction peptidique héminique soluble précitée, après dessalage éventuel, et le rétentat d'ultrafiltration constituant la fraction peptidique héminique insoluble précitée, après purification éventuelle.

La présente invention porte sur les fractions obtenues ainsi que sur leurs applications. Ces fractions trouvent en effet application chaque fois qu'il convient de réaliser un apport en fer, en médecine humaine et vétérinaire, par exemple pour l'administration à des femmes venant d'accoucher et présentant, de ce fait, une carence en fer.

Les hydrolysats de protéines sont maintenant largement utilisés en tant qu'aliments de nutrition pour l'alimentation humaine et animale, et également dans des applications particulières, dans des domaines tels que la nutrition entérale clinique, les produits vétérinaires, et l'isolement des peptides à activité biologique. Des recherches sont menées de façon croissante dans le domaine de la thérapeutique nutritionnelle, où l'on demande des hydrolysats présentant une pureté élevée et une constance de composition.

Jusqu'à maintenant, la plupart des hydrolysats de protéines étaient obtenus par un traitement enzymatique de sources de protéines, telles que le soja, la caséine, le blé et le poisson.

Le sang destiné à l'utilisation en alimentation humaine est généralement centrifugé de façon à séparer le plasma (composé de protéines incolores) du cruor, qui est le résidu cellulaire solide constitué à près de 95% d'hémoglobine, protéine facile à obtenir à l'état pur et ayant une composition constante d'un lot à l'autre. Alors que le plasma est utilisé notamment dans les industries de salaison et de conserve de viandes, la quasi-totalité du cruor est rejetée à titre de déchets, ou très faiblement valorisée, par exemple, traitée pour entrer dans la composition d'engrais, de colles, etc.

On a donc cherché à valoriser le cruor, afin d'obtenir des fractions peptidiques pouvant être utilisées dans l'alimentation animale ainsi que dans l'alimentation humaine, par exemple par incorporation dans les produits de charcuterie ou dans les potages fabriqués industriellement, ou encore en diététique médicale, par incorporation dans des formulations alimentaires diététiques, notamment pour les nourrissons.

On a déjà proposé un procédé discontinu d'obtention de globine décolorée hydrolysée à propriétés fonctionnelles intéressantes (pouvoir d'émulsification ; propriétés moussantes), supérieures à celles du caséinate, suivant lequel on réalise une hydrolyse enzymatique du cruor, en cuve agitée, l'enzyme étant l'Alcalase® ; on inactive l'enzyme par chauffage et à pH acide ; on sépare, par centrifugation ou ultrafiltration, la fraction fortement colorée précipitée, d'une fraction liquide moins colorée, que l'on traite ensuite par le charbon actif ; on concentre le produit liquide clair obtenu par les techniques classiques d'élimination de l'eau (osmose inverse ou évaporation) ; puis on le stabilise et on le sèche par atomisation.

On a aussi proposé, dans la demande de brevet français n° 2 561 074, de réaliser une hydrolyse enzymatique d'un hémolysat de cruor. Le procédé est un procédé en discontinu mettant en jeu une enzyme protéolytique digestive, comme la pepsine, après quoi, on centrifuge et on décolore le surnageant par mise en contact avec un agent adsorbant choisi parmi l'alumine, la magnésie et le silicate de magnésium. La fraction peptidique obtenue après lyophilisation du produit ainsi décoloré ne présente plus d'activité enzymatique et a une valeur nutritive intéressante. Ce procédé n'est cependant pas avantageusement applicable à l'échelle industrielle.

On a maintenant découvert que l'hydrolyse en continu d'un hémolysat du cruor, dans un réacteur enzymatique à ultrafiltration sur membranes minérales, pouvait conduire à des fractions peptidiques héminiques de valeur, dans des conditions industriellement satisfaisantes. Il n'était pas évident que la technique connue d'hydrolyse dans un réacteur enzymatique à ultrafiltration s'applique aux hémolysats du cruor. On pouvait en effet s'attendre à un colmatage des membranes d'ultrafiltration par suite d'une accumulation dans le circuit de peptides insolubles riches en hème. Par ailleurs, on obtient, selon l'invention, un perméat d'ultrafiltration nettement moins coloré que l'hydrolysat enzymatique brut, mais contenant encore suffisamment d'hème pour présenter de l'intérêt en thérapeutique pour réaliser un apport en fer, en raison de la solubilité de cette fraction.

Par ailleurs, avec le procédé selon l'invention, en agissant sur différents paramètres, en particulier les paramètres du réacteur enzymatique à ultrafiltration (débit, pression), on peut obtenir des hydrolysats et, après purification, des fractions peptidiques qui sont mieux adaptés aux besoins et dont on peut contrôler la reproductibilité et la pureté.

Egalement, le rétentat d'ultrafiltration a été caractérisé en ce qui concerne sa teneur en fer, ce qui a permis de mettre en évidence son utilité dans des applications thérapeutiques nécessitant un apport en fer.

La présente invention a donc d'abord pour objet un procédé de préparation de fractions peptidiques héminiques à partir d'un hémolysat du cruor, caractérisé par le fait qu'il consiste à :
- introduire ledit hémolysat dans une zone de réaction, en vue d'une mise en contact intime avec une enzyme protéolytique travaillant à un pH inférieur à 4 ;
- soutirer en continu le produit de la réaction enzymatique en l'amenant de la zone de réaction dans une zone d'ultrafiltration comportant des membranes minérales d'ultrafiltration ayant un seuil de coupure leur permettant de retenir ladite enzyme et l'hémoglobine, laissant passer pratiquement seulement les peptides provenant de l'hydrolyse ;
- soutirer en continu un perméat qui représente une première fraction peptidique héminique recherchée (fraction F1) ;
- recycler, au moins partiellement, le rétentat d'ultrafiltration dans la zone de réaction ; et
- recueillir le rétentat, en tant que seconde fraction peptidique héminique recherchée (fraction F2).

On utilise notamment un hémolysat de cruor ayant une teneur en hémoglobine comprise entre 1 et 10% en poids, le cruor ayant été hémolysé en milieu aqueux, à un pH acide, notamment de 2, puis dilué jusqu'à la teneur en hémoglobine recherchée.

En particulier, on choisit, comme enzyme protéolytique, la pepsine, et on ajuste le pH dans la zone de réaction à une valeur allant de 1,5 à 2,5, par addition d'un composé à réaction acide. On introduit la pepsine dans la zone de réaction à raison de 40 à 80 unités ANSON par kilogramme d'hémoglobine (l'unité ANSON étant définie dans ≪J. GEN. PHYSIOL. 22, 7989-1939≫). Par ailleurs, on effectue l'hydrolyse à une température inférieure à la température de dénaturation de l'enzyme utilisée ; on choisira notamment une température se situant dans la zone de 35 à 45° C.

Conformément à une autre caractéristique de l'invention, on met en circulation le milieu se trouvant dans la zone de réaction, alors que le taux d'hydrolyse est de l'ordre de 10 à 20%.

On choisit des membranes minérales présentant un seuil de coupure inférieur à 30 000 environ, pouvant être aussi faible que 10 000 environ, et même pouvant présenter une valeur inférieure à 10 000. A titre d'exemple de membranes d'ultrafiltration, on peut mentionner les membranes tubulaires de carbone recouvertes d'oxyde de zirconium, telles que celles mises initialement sur le marche par la Société de Fabrication d'Eléments Catalytiques (SFEC), France.

Ce procédé se distingue de celui décrit dans la demande de brevet européen n° 0 061 556 qui vise seulement la préparation de peptides héminiques. Ce procédé connu met en jeu une étape de dénaturation à pH 11 de l'hémoglobine, avant de conduire l'hydrolyse enzymatique à un pH se situant entre 9 et 7, l'enzyme employée étant notamment l'Alcalase®, la neutrase ou la papaïne, mais en tout cas, jamais la pepsine. Selon l'invention, la pepsine est mise en oeuvre à son pH optimum de 2 qui est dénaturant pour l'hémoglobine. Les membranes organiques employés conformément au procédé de la demande européenne n° 0 061 556 pourraient être difficilement mises en oeuvre à une telle valeur de pH.

Dans ces conditions, les fractions peptidiques de la présente invention auront donc nécessairement une composition distincte des produits obtenus par ce procédé connu. Le procédé de l'invention permet de préparer en continu des hydrolysats peptidiques définis (répartition en masses moléculaires et composition en acides aminés) et reproductibles à l'abri des contaminations bactériennes, rendant ainsi les peptides applicables dans des domaines où cette reproductibilité est importante, comme en diététique médicale.

L'ultrafiltrat obtenu par le procédé selon l'invention peut être dessalé, en utilisant pour cela, à titre d'exemple, la technique d'électrodialyse. Ce dessalage conduit à une fraction peptidique héminique F3.

On peut ensuite concentrer et atomiser l'ultrafiltrat, éventuellement dessalé, pour obtenir la fraction peptidique recherchée (F1 ou F3) sous la forme d'une poudre. En particulier, on peut réaliser l'opération de concentration à l'aide d'un évaporateur à flot tombant.

Conformément à la présente invention, on peut conduire une précipitation à pH acide, inférieur à environ 7, du rétentat, puis on filtre, ce qui conduit, d'une part, à une fraction peptidique héminique insoluble F4 contenant la quasi-intégralité du fer héminique présent dans le rétentat, et, d'autre part, à une fraction peptidique héminique soluble F5.

On peut aussi conduire une centrifugation du rétentat brut, ce qui conduit, d'une part, à une fraction peptidique héminique insoluble F'4, et, d'autre part, à une fraction peptidique héminique soluble F'5.

Enfin, on peut effectuer les opérations suivantes:
- introduire ledit rétentat dans une zone de réaction en vue d'une mise en contact intime avec une enzyme protéolytique travaillant à un pH supérieur à 5 ;
- soutirer, le cas échéant en continu, le produit de la réaction enzymatique en l'amenant de la zone de réaction dans une zone d'ultrafiltration comportant des membranes d'ultrafiltration ayant un seuil de coupure leur permettant de retenir ladite enzyme ;
- recueillir le rétentat et le concentrer ; et
- conduire une précipitation à pH acide pour obtenir, d'une part, une fraction peptidique héminique insoluble F6, et, d'autre part, une fraction peptidique héminique soluble F7.

En particulier, on choisit, comme enzyme protéolytique, la neutrase, l'Alcalase®, la papaïne ou la pancréatine, et on ajuste le pH à respectivement 6, 9, 7 et 8. On introduit l'enzyme dans la zone de réaction à raison d'environ 0,01 à 0,15% (p/v). Par ailleurs, on effectue avantageusement les hydrolyses à respectivement environ 40°C, 60°C, 60°C et 40°C. On choisit, par exemple, des membranes organiques d'ultrafiltration Sartorius® de seuil de coupure 10 000 D.

Les fractions héminiques solubles selon l'invention sont utiles notamment dans le traitement de l'anémie.

La présente invention porte également sur la fraction peptidique héminique soluble qui consiste en le perméat d'ultrafiltration d'un hydrolysat peptidique de cruor, ladite hydrolyse ayant été conduite en milieu acide à pH 1,5-2,5 par une enzyme protéolytique, ladite fraction possédant une teneur en fer au moins égale à 0,25 mg par g de matières sèches. L'invention porte également sur la fraction peptidique précitée, pour laquelle l'hydrolyse a été suivie d'un dessalage, ladite fraction possédant alors une teneur en fer au moins égale à 0,5 mg par g de matières sèches.

L'invention porte aussi sur une fraction peptidique héminique insoluble, caractérisée par le fait qu'elle consiste en le rétentat d'ultrafiltration d'un hydrolysat peptidique de cruor, ladite hydrolyse ayant été conduite en milieu acide à pH 1,5-2,5 par une enzyme protéolytique, le rétentat ayant été soumis à une centrifugation ou à une précipitation à pH acide, la teneur en fer de ladite fraction étant d'au minimum 1% en poids des matières sèches ; l'invention porte aussi sur une fraction peptidique héminique insoluble résultant d'un traitement d'hydrolyse par une enzyme protéolytique travaillant à un pH supérieur à 5 (comme l'Alcalase®), avec ultrafiltration, du rétentat tel que défini ci-dessus, ce traitement ayant été suivi par une précipitation à pH acide, la fraction précipitée possédant alors une teneur en fer au moins égale à 30 mg par g de matières sèches.

La présente invention porte enfin sur une composition pharmaceutique destinée à réaliser un apport en fer en thérapeutique humaine et animale, caractérisée par le fait qu'elle renferme, comme ingrédient actif, une fraction peptidique héminique obtenue par le procédé tel que défini ci-dessus, ou telle que définie ci-dessus ; ainsi que sur des formes pharmaceutiques convenant pour l'administration orale, entérale ou par voie injectable, renfermant, à titre d'ingrédient actif, les fractions peptidiques héminiques solubles obtenues par le procédé tel que défini ci-dessus, ou les fractions peptidiques héminiques solubles telles que définies ci-dessus, ainsi que sur des formes pharmaceutiques convenant pour l'administration orale, entérale ou par voie injectable, renfermant, à titre d'ingrédient actif, les fractions peptidiques héminiques insolubles obtenues par le procédé tel que défini ci-dessus, ou les fractions peptidiques héminiques insolubles telles que définies ci-dessus.

Dans le cas de la fraction peptidique héminique insoluble utilisable en thérapie humaine et animale chaque fois qu'il est nécessaire de réaliser un apport en fer, on peut mentionner une forme pharmaceutique intéressante qui est constituée par les gélules.

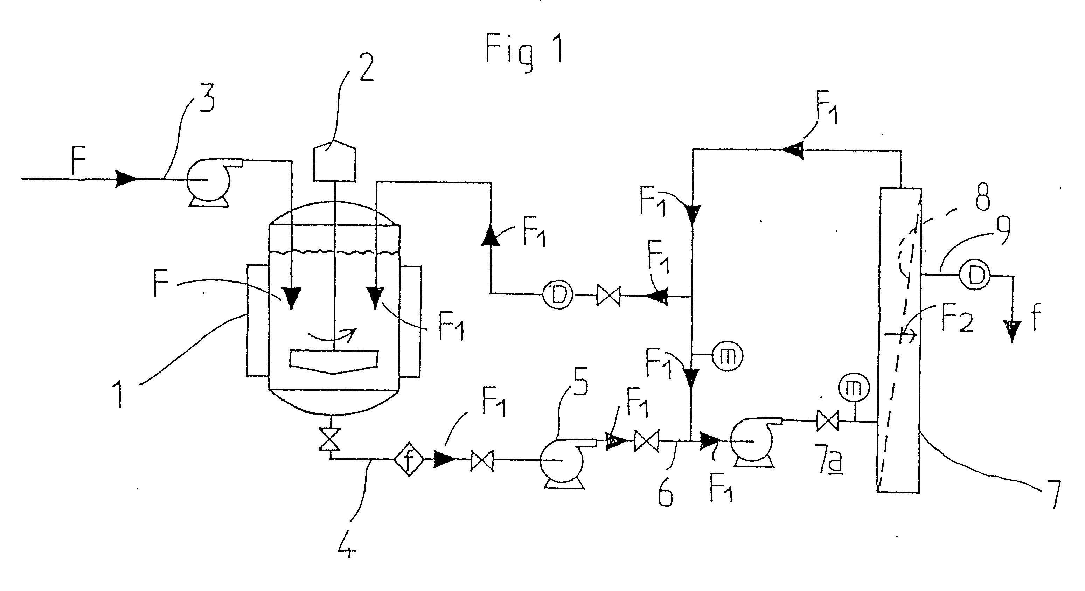

On a représenté, sur la Figure 1 du dessin annexé, le réacteur enzymatique à membranes minérales, utilisé pour l'hydrolyse du cruor. Sur ce dessin, (m) désigne un manomètre, chaque (D), un débitmètre, et (f), un filtre.

Ce réacteur enzymatique comporte une cuve à réaction désignée par le chiffre de référence 1. Cette cuve 1 est équipée d'un système d'agitation 2. L'alimentation continue en hémolysat est effectuée par la canalisation 3, comme indiqué par la flèche F. La pepsine est introduite directement dans la cuve 1. Cette dernière est également équipée d'un appareillage servant à la fois à mesurer et à maintenir le pH dans la cuve de réaction. Le produit d'hydrolyse prélevé dans la cuve de réaction 1 par la canalisation 4 est acheminé par une pompe d'alimentation ou de gavage 5. Au refoulement de la pompe 5, le produit passe dans une canalisation 6 et est amené dans un module d'ultrafiltration 7. Ce module 7 comprend une pompe de circulation 7a et des membranes tubulaires 8 dont les caractéristiques sont indiquées ci-après pour chacun des deux exemples de mise en oeuvre du procédé de l'invention.

La circulation du produit d'hydrolyse renfermant l'enzyme est symbolisée par les flèches $F_1$, et le passage de la fraction peptidique d'ultrafiltrat à travers les membranes 8, par la flèche $F_2$.

L'ultrafiltrat est récupéré dans une canalisation 9 (comme indiqué par la flèche f). Quant au rétentat du module 7, il est prélevé par la canalisation 10, sur laquelle peut se trouver un échangeur, et il est acheminé par cette canalisation pour être recyclé au moins partiellement dans la cuve de réaction 1.

Grâce à l'utilisation d'un réacteur enzymatique combiné à une ultrafiltration sur membranes minérales, on peut hydrolyser l'hémoglobine en continu.

Dans ce qui suit, on a décrit deux exemples illustrant la mise en oeuvre du procédé complet selon la présente invention ; le mode opératoire général était le même dans les deux cas, mais les membranes utilisées étaient différentes.

## A. HYDROLYSE ENZYMATIQUE CONTINUE

On a obtenu l'hémoglobine en effectuant une hémolyse d'érythrocytes par de l'eau, et on en a déterminé la concentration conformément à la méthode à la cyanméthémoglobine (Crosby, W. H. ; Munn, J. I. ; Furth, F. W. «Standardizing a method for clinical hemoglobinometry. U. S. Armed Forces Med. J. 1954, 5, 693 - 703»), afin d'obtenir 300 l d'hémoglobine dénaturée à 5% (p/v), à pH 2. Pour la digestion pepsique, on a chauffé 80 l d'hémoglobine jusqu'à 40°C dans un réacteur (SETRIC), et on a ajouté 240 Unités ANSON de pepsine porcine (MERCK). L'hydrolyse a duré 8 heures, à un pH de 2, maintenu par le dispositif pH statique (Electrode INGOLD type 465 dans une sonde de type 764), délivrant de l'acide chlorhydrique 4,2 M. Des échantillons (5 ml) ont été prélevés du milieu d'incubation, et la teneur en azote a été déterminée conformément à la méthode de KJELDHAL (appareil GERHARDT VAPODEST 3). La teneur en protéines a été exprimée sous forme N x 6,25.

Au bout de 8 heures, le réacteur a été relié aux membranes d'ultrafiltration. Le dispositif d'ultrafiltration était équipé de deux modules, en séries, de membranes M4 (Exemple 1) ou M5 (Exemple 2) Carbosep SFEC, d'une pompe d'alimentation (HILGE type Hygiana 1/.3, type centrifuge : débit 1 m³/h, 5 bars) et d'une pompe de circulation (HILGE type Hygia super II, type centrifuge : débit 5 m³/h, 1,8 bars). Chaque module présentait une surface de 0,16 m² et se composait de sept tubes mesurant chacun 1,2 m de longueur, avec un diamètre interne de 6 mm et un diamètre externe de 10 mm ; les tubes étaient réalisés en carbone poreux et revêtus par une couche active d'oxyde de zirconium sur leur surface interne. Des membranes présentant des seuils de coupure de respectivement 10 000 daltons (membranes M5 de l'Exemple 2) et 20 000 daltons (membrane M4 de l'Exemple 1) ont été utilisées. La résistance physique et chimique des membranes et l'appareillage permettaient une opération dans une plage de températures de 0 - 85°C, dans une plage de pH 1 - 14, et dans une plage de pressions de 0 - 8 bars. Le débit de perméation caractéristique pour l'eau était de 60 l/h/m²/bar (pour les membranes M4) et de 45 l/h/m²/bar (pour les membranes M5) à 25°C.

L'ultrafiltration a été conduite à 40°C pendant 22 heures. Les pressions de travail ont été ajustées manuellement, afin de maintenir constant le débit de l'ultrafiltrat (30 l/m²/h). La vitesse d'écoulement transversal a été fixée à 4 m/s. Le réacteur était équipé d'un régulateur de niveau. Pendant l'ultrafiltration, le niveau du réacteur a été maintenu constant par l'addition d'hémolysat.

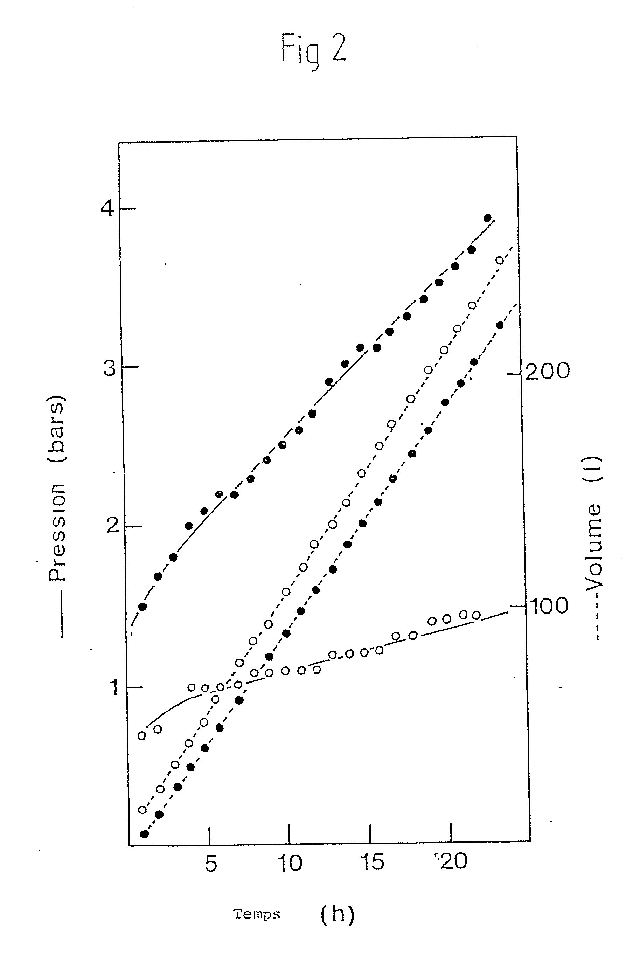

Les pressions internes des membranes ont été suivies en fonction du temps, à un débit de perméat de 10 l/h pour les membranes M4 (Exemple 1) et M5 (Exemple 2) ; c'est ce qui est représenté sur la Figure 2 :

**Légende de la Figure 2 :** Effet de la sélectivité des membranes sur les pressions internes des membranes et les volumes de perméat en fonction du temps. Ultrafiltration avec membranes M4 (o) et membranes M5 (.).

Les pressions internes des membranes passent de 1 bar à 1,4 bar, et de 1,5 bar à 4 bars pour les membranes respectivement M4 et M5.

## B. ELECTRODIALYSE DE l'ULTRAFILTRAT

Le dessalage de l'ultrafiltrat a été effectué par électrodialyse (Aqualizer P20 de SRTI). On a contrôlé l'élimination du sel en suivant l'élimination de l'intensité (8 à 0,5 A), sous une tension constante imposée (110 V).

## C. CONCENTRATION ET ATOMISATION DE L'ULTRAFILTRAT

L'ultrafiltrat dessalé a été d'abord concentré à l'aide d'un évaporateur (FIVRS CAIL), puis atomisé (Atomiseur NIRO). La concentration a été effectuée à 78°C, sous 0,5 bar. L'atomisation a été effectuée entre 200 et 100°C.

La même opération a été répétée sur de l'ultrafiltrat non dessalé.

Les valeurs des rendements des différentes étapes du procédé selon les Exemples 1 et 2 sont rapportées dans le Tableau 1. Les quantités de protides sont exprimées sous forme N x 6,25.

Tableau 1 :

| Etapes | Exemple 1 | | Exemple 2 | |
|---|---|---|---|---|
| | Quantité de protides récupérée | Rendement | Quantité de protides récupérée | Rendement |
| | (kg) | (%) | (kg) | (%) |
| Hémolyse | 11,1 | 100 | 11,7 | 100 |
| Ultrafiltration | 8,6 | 77 | 8,1 | 69 |
| Dessalage | 7,6 | 88 | 6,5 | 80 |
| Concentration | 6,6 | 88 | 6,3 | 98 |
| Atomisation | 6,5 | 97 | 6,2 | 98 |
| Rendements totaux | | 58 | | 53 |

## D. ANALYSE CHIMIQUE ELEMENTAIRE DE L'ULTRAFILTRAT

Les résultat de l'analyse chimique élémentaire des poudres peptidiques obtenus à l'Exemple 1 (Membranes M4) sont rapportés dans le Tableau 2 ci-après. Les quantités d'éléments sont exprimées en g pour 100 g de poudre.

Tableau 2

| Eau et Eléments | ULTRAFILTRAT (sur M4) | |
|---|---|---|
| | Non dessalé (%) | Dessalé (%) |
| N | 12,35 | 13,92 |
| $H_2O$ | 7,50 | 8,03 |
| Na | 1,89 | 0,12 |
| Mg | $8,0 \times 10^{-3}$ | $1,4 \times 10^{-2}$ |
| Cl | 11,06 | 1,79 |
| Ca | $4,7 \times 10^{-2}$ | $5,3 \times 10^{-2}$ |
| P | $4,4 \times 10^{-2}$ | $4,8 \times 10^{-2}$ |
| Fe | $2,5 \times 10^{-2}$ | $5,7 \times 10^{-2}$ |
| K | 0,15 | $1,5 \times 10^{-3}$ |
| Cu | $5,0 \times 10^{-4}$ | $5,0 \times 10^{-4}$ |
| Pb | $3,5 \times 10^{-4}$ | $2,0 \times 10^{-4}$ |
| Cd | $1,0 \times 10^{-6}$ | $3,5 \times 10^{-6}$ |

La teneur en azote permet d'évaluer le pourcentage de peptides dans la poudre peptidique comme étant un pourcentage dépassant 90% (N x 6,25).

Arès dessalage, la concentration en fer dans cet ultrafiltrat est d'environ 57 mg pour 100 g de matières sèches. Il s'agit de fer héminique totalement soluble. Un tel ultrafiltrat permet donc un apport à la fois de fer soluble et de protides solubles.

## E. RETENTAT

La détermination de la teneur en fer du rétentat brut par une méthode de spectrométrie atomique permet de conclure que le fer représente 1% de la matière sèche du rétentat.

Afin de pouvoir déterminer la quantité d'insolubles dans le rétentat, c'est-à-dire de peptides susceptibles de porter des hèmes, on amène le pH du rétentat brut à différentes valeurs, afin de précipiter ces insolubles, puis on le filtre sur un filtre de porosité 0,45 um. Les résultats sont les suivants :

| pH | 2 | 3 | 4 | 6 | 7 | 8 | 9 |
|---|---|---|---|---|---|---|---|
| % pondéral des insolubles | 0,72 | 0,68 | 0,74 | 0,72 | 0,70 | 0,43 | 0,40 |

La solubilité des composants du rétentat est fonction du pH. On constate que, pour récupérer le plus d'insolubles, il faut précipiter le rétentat à des pH acides et récupérer ainsi le maximum d'hème qui est porté par ces peptides insolubles. Il y a en fait aggrégation des peptides héminiques entre eux (l'hème est insoluble pour des pH $\leq$ 4,5).

Le taux de fer dans ces peptides héminiques insolubles est déterminé par un dosage spectrophotométrique mettant en oeuvre le 2,4,6-tri-(2-pyridyl)S-triazine (T.P.T.Z.). Le rétentat ayant été précipité à différents pH acides et filtré, on effectue le dosage des insolubles :

| pH | 1,5 | 2 | 2,5 | 3 |
|---|---|---|---|---|
| % de fer dans les insolubles | 1,01 | 1,07 | 1,09 | 1,12 |

Pour enrichir le rétentat en fer, on conduit une hydrolyse enzymatique à l'Alcalase® du rétentat brut dans un module d'ultrafiltration Sartorius® (réf. SM 16526) sous une pression d'azote de 2 bars sur un filtre de seuil de coupure en poids moléculaire de 10 000 Dalton Sartorius® (réf. 14539).

Cette hydrolyse s'effectue à 60°C et à pH non constant (pH de départ = 11) sur 100 ml de rétentat. Le rétentat est concentré à 10 ml sous une pression d'azote de 2 bars. En raison de sa viscosité, ce concentré ne peut être filtré sur des filtres de 0,45 um. Il est donc centrifugé après acidification à 20 000 tours pendant 30 minutes, puis le culot qui renferme les insolubles est lyophilisé, afin de déterminer le poids sec des insolubles. Ceux-ci sont ensuite déminéralisés, afin de déterminer leur teneur en fer.

Résultats- Volume de rétentat hydrolysé et concentré final : 10 ml
- Teneur en fer des insolubles : 3,6%.

L'augmentation de la teneur en fer du rétentat est donc très importante. Il semblerait donc que l'élimination au fur et à mesure des petits peptides non héminiques hydrolysés induise une hydrolyse plus poussée ; cette élimination pourrait en effet lever une inhibition due à la présence de nombreux peptides.

## Revendications

1 - Procédé de préparation de fractions peptidiques héminiques à partir d'un hémolysat du cruor, caractérisé par le fait qu'il consiste à :
- introduire ledit hémolysat dans une zone de réaction (1), en vue d'une mise en contact intime avec une enzyme protéolytique travaillant à un pH inférieur à 4 ;
- soutirer en continu le produit de la réaction enzymatique en l'amenant de la zone de réaction (1) dans une zone d'ultrafiltration (7) comportant des membranes minérales d'ultrafiltration (8) ayant un seuil de coupure leur permettant de retenir ladite enzyme et l'hémoglobine, laissant passer pratiquement seulement les peptides provenant de l'hydrolyse ;
- soutirer en continu un perméat qui représente une fraction peptidique héminique recherchée (fraction F1);

- recycler, au moins partiellement, le rétentat d'ultrafiltration dans la zone de réaction (1) ; et

- recueillir le rétentat, en tant que seconde fraction peptidique héminique recherchée (fraction F2).

2 - Procédé selon la revendication 1, caractérisé par le fait qu'on utilise un hémolysat de cruor ayant une teneur en hémoglobine comprise entre 1 et 10% en poids, le cruor ayant été hémolysé en milieu aqueux, à un pH acide, notamment de 2, puis dilué jusqu'à la teneur en hémoglobine recherchée.

3 - Procédé selon l'une des revendications 1 et 2, caractérisé par le fait qu'on choisit la pepsine, comme enzyme protéolytique, et qu'on ajuste le pH dans la zone de réaction (1) à une valeur allant de 1,5 à 2,5, par addition d'un composé à réaction acide.

4 - Procédé selon la revendication 3, caractérisé par le fait qu'on introduit la pepsine dans la zone de réaction (1), à raison de 40 à 80 unités ANSON par kilogramme d'hémoglobine.

5 - Procédé selon l'une des revendications 1 à 4, caractérisé par le fait qu'on effectue l'hydrolyse à une température de l'ordre de 35-45° C.

6 - Procédé selon l'une des revendications 1 à 5, caractérisé par le fait qu'on met en circulation le milieu se trouvant dans la zone de réaction (1), alors que le taux d'hydrolyse est de l'ordre de 10 à 20 %.

7 - Procédé selon l'une des revendications 1 à 6, caractérisé par le fait qu'on choisit des membranes minérales présentant un seuil de coupure inférieur à 30 000 environ.

8 - Procédé selon l'une des revendications 1 à 7, caractérisé par le fait qu'on utilise, comme membranes d'ultrafiltration, des membranes tubulaires de carbone recouvertes d'oxyde de zirconium.

9 - Procédé selon l'une des revendications 1 à 8, caractérisé par le fait qu'on dessale le perméat, ce qui conduit à une fraction peptidique héminique F3.

10 - Procédé selon la revendication 9, caractérisé par le fait qu'on effectue le dessalage par électrodialyse.

11 - Procédé selon l'une des revendications 1 à 10, caractérisé par le fait qu'après dessalage éventuel, on concentre et on atomise le perméat pour obtenir la fraction recherchée (F1 ou F3) sous forme de poudre.

12 - Procédé selon la revendication 11, caractérisé par le fait qu'on concentre à l'aide d'un évaporateur à flot tombant.

13 - Procédé selon l'une des revendications 1 à 12, caractérisé par le fait qu'on conduit une précipitation à pH acide, inférieur à environ 7, du rétentat, puis on filtre, ce qui conduit, d'une part, à une fraction peptidique héminique insoluble F4 contenant la quasi-intégralité du fer héminique présent dans le rétentat, et, d'autre part, à une fraction peptidique héminique soluble F5.

14 - Procédé selon l'une des revendications 1 à 12, caractérisé par le fait qu'on conduit une centrifugation du rétentat brut, ce qui conduit, d'une part, à une fraction peptidique héminique insoluble F'4, et, d'autre part, à une fraction peptidique héminique soluble F'5.

15 - Procédé selon l'une des revendications 1 à 12, caractérisé par le fait qu'il comprend les étapes supplémentaires consistant à :

- introduire ledit rétentat dans une zone de réaction en vue d'une mise en contact intime avec une enzyme protéolytique travaillant à un pH supérieur à 5 ;

- soutirer le produit de la réaction enzymatique en l'amenant de la zone de réaction dans une zone d'ultrafiltration comportant des membranes d'ultrafiltration ayant un seuil de coupure leur permettant de retenir ladite enzyme ;

- recueillir le rétentat et le concentrer ;

- conduire une précipitation à pH acide pour obtenir, d'une part, une fraction peptidique héminique insoluble F6, et, d'autre part, une fraction peptidique héminique soluble F7.

16 - Procédé selon la revendication 15, caractérisé par le fait qu'on utilise, comme enzyme, la neutrase, l'Alcalase, la papaïne ou la pancréatine.

17 - Fraction peptidique héminique soluble, caractérisée par le fait qu'elle consiste en le perméat d'ultrafiltration d'un hydrolysat peptidique de cruor, ladite hydrolyse ayant été conduite en milieu acide à pH 1,5-2,5 par une enzyme protéolytique, ladite fraction possédant une teneur en fer au moins égale à 0,25 mg par g de matières sèches.

18 - Fraction peptidique héminique soluble, caractérisée par le fait qu'elle consiste en le perméat d'ultrafiltration d'un hydrolysat peptidique de cruor, ladite hydrolyse ayant été conduite en milieu acide à pH 1,5-2,5 par une enzyme protéolytique, et ayant été suivie par un dessalage, ladite fraction possédant alors une teneur en fer au moins égale à 0,5 mg par g de matières sèches.

19 - Fraction peptidique héminique insoluble, caractérisée par le fait qu'elle consiste en le rétentat d'ultrafiltration d'un hydrolysat peptidique de cruor, ladite hydrolyse ayant été conduite en milieu acide à pH 1,5-2,5 par une enzyme protéolytique, la teneur en fer de ladite fraction étant d'au minimum 1% en poids des matières sèches.

20 - Fraction peptidique héminique insoluble, caractérisée par le fait qu'elle résulte d'un traitement d'hydrolyse par une enzyme travaillant à pH supérieur à 5, avec ultrafiltration, du rétentat tel que défini à la revendication 19, ce traitement ayant été suivi par une précipitation à pH acide, la fraction précipitée possédant alors une teneur en fer au moins égale à 30 mg par g de matières sèhes.

21 - Composition pharmaceutique destinée à réaliser un apport en fer en thérapeutique humaine et animale, caractérisée par le fait qu'elle renferme, comme ingrédient actif, une fraction peptidique héminique obtenue par le procédé tel que défini à l'une des revendications 1 à 16, ou telle que définie à l'une des revendications 17 à 20.

22 - Formes pharmaceutiques convenant pour l'administration orale, entérale ou par voie injectable, renfermant, à titre d'ingrédient actif, les fractions peptidiques héminiques solubles obtenues par le procédé tel que défini à l'une des revendications 1 à 16, ou les fractions peptidiques héminiques solubles telle que définies aux revendications 17 et 18.

23 - Formes pharmaceutiques convenant pour l'administration orale, entérale ou par voie injectable, renfermant, à titre d'ingrédient actif, les fractions peptidiques héminiques insolubles obtenues par le procédé tel que défini à l'une des revendications 1 à 16, ou les fractions peptidiques héminiques insolubles telles que définies aux revendications 19 et 20.

Fig 1

EP 0 436 747 A1

# Fig 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | JOURNAL CHEM. TECH. BIOTECHNOLOGY, vol. 42, no. 2, 1988, Seiten 147-156; J.-M. PIOT et al.: "Application of ultrafiltration to the preparation of defined hydrolysates of bovine haemoglobin"<br>* En entier *<br>--- | 1-14,17 -19,21- 23 | C 12 P 21/06<br>A 61 K 37/18<br>A 23 J 1/06 |
| Y | IDEM<br><br>--- | 15-16, 20 | |
| Y | ACM SYMP. SERIES, no. 47, 1977, pages 295-303; K.J. STACHOWICZ et al.: "Enzymic hydrolysis of ox-blood hemoglobin"<br>* En entier *<br>--- | 15-16, 20 | |
| A,D | EP-A-0 061 556 (AB PRIPPS BRYGGERIER)<br>* Pages 9-12 *<br>--- | 1 | |
| A | MIRCEN JOURNAL, vol. 2, 1986, pages 359-364, Oxford University Press; J.M. PIOT et al.: "Preparation of decolorized peptides from slaughter-house blood"<br>* Pages 359-360 *<br>--- | 1 | DOMAINES TECHNIQUES RECHERCHES (Int. Cl.5)<br><br>C 12 P<br>A 61 K<br>A 23 J |
| A | WO-A-8 202 841 (UNIVERSITY PATENTS INC.)<br>--- | 1-5 | |
| E | EP-A-0 354 100 (CIBEVIAL)<br>* Exemples 1-4 *<br>----- | | |

Le présent rapport a été établi pour toutes les revendications

| Lieu de la recherche | Date d'achèvement de la recherche | Examinateur |
|---|---|---|
| LA HAYE | 03-05-1990 | ALVAREZ Y ALVAREZ C. |

EPO FORM 1503 03.82 (P0402)